# FASCICULE DE BREVET EUROPEEN

(11) **EP 0 692 248 B1**
(45) Date de publication et mention de la délivrance du brevet: **10.01.2001**
(21) Numéro de dépôt: 95401205.0
(22) Date de dépôt: 23.05.1995
(51) Int. Cl.: A61K 7/50, A61K 7/00, A61K 7/13, A61K 7/06, A61K 7/09

(54) **Composition capillaire solide contenant un agent structurant particulaire**
Eine teilchenförmige strukturgebende Substanz enthaltendes festes Haarpflegemittel
Solid hair treating composition containing a particulated structurant

(30) Priorité: 11.07.1994 FR 9408567
(43) Date de publication de la demande: 17.01.1996
(73) Titulaire: L'OREAL, 75008 Paris (FR)
(72) Inventeur: Dubief, Claude, F-78150 Le Chesnay (FR); De La Mettrie, Roland, F-78110 Le Vesinet (FR); De Labbey, Arnaud, F-93600 Aulnay Sous Bois (FR); Rondeau, Christine, F-78500 Sartrouville (FR)
(74) Mandataire: Lhoste, Catherine

(56) Documents cités:
- EP-A- 0 214 626
- EP-A- 0 287 773
- EP-A- 0 295 903
- EP-A- 0 348 015
- EP-A- 0 379 409
- EP-A- 0 486 394
- WO-A-94/14402

## Description

La présente invention a pour objet une nouvelle composition capillaire ayant un aspect de solide déformable. Cette composition capillaire peut consister en un shampooing, un après shampooing, une composition pour colorer ou décolorer les fibres kératiniques ou encore une composition pour permanenter ou défriser ces fibres. L'invention s'applique plus spécialement aux traitements des cheveux, des cils ou sourcils des êtres humains.

L'invention se rapporte aussi à un procédé de traitement cosmétique des fibres kératiniques, à un procédé pour colorer, décolorer, permanenter, laver et/ou conditionner les fibres kératiniques ainsi qu'à un procédé pour délivrer un actif particulier sur les fibres kératiniques.

L'invention se rapporte encore à l'utilisation d'un agent structurant particulier dans une composition capillaire.

Les compositions capillaires se présentent le plus souvent sous forme de liquides ou crèmes plus ou moins visqueux. Plus les compositions sont liquides, plus il est difficile de les prendre dans les mains et plus il est difficile de les doser. En effet, ces compositions ont tendance à s'échapper entre les doigts. Par ailleurs, plus ces compositions sont liquides, plus elles ont tendance à s'échapper de leur conditionnement.

On connait dans les documents EP-A-348 015, EP-A-295 903 et EP-A-379 409 des compositions pateuses contenant des particules solides. Aucun de ces documents ne décrit de compositions solides déformables.

De façon simplifiée, une composition capillaire comprend un ou plusieurs actifs tels que des détergents, des colorants, des actifs conditionneurs, des actifs de permanente, dans un support ou milieu cosmétiquement acceptable contenant une grande quantité d'eau et le plus souvent des agents tensioactifs.

Après chaque traitement spécifique des cheveux (shampooing, permanente, coloration etc.), il est généralement nécessaire de rincer les cheveux afin de ne garder sur ceux-ci que les actifs traitants (conditionneur, colorant) et éliminer le support et notamment les agents tensioactifs. Malheureusement, un grand nombre de compositions capillaires présente l'inconvénient d'être difficile à rincer, c'est-à-dire long à rincer, et/ou de laisser des traces de produit sur les cheveux leur conférant notamment un aspect collant, ciré, poisseux.

Par ailleurs, les utilisateurs recherchent, de plus en plus, de nouvelles textures et de nouveaux concepts de produits.

La présente invention a justement pour objet une nouvelle composition capillaire permettant notamment de remédier aux inconvénients mentionnés ci-dessus. En particulier cette composition se rince de façon remarquable et présente une texture tout à fait inhabituelle. En outre, elle est simple à appliquer.

Par "capillaire", il faut comprendre aussi bien les cheveux que les cils et les sourcils.

De façon surprenante, la demanderesse a trouvé qu'il était possible de conférer à une composition capillaire un aspect de solide déformable en utilisant un agent structurant ou texturant original.

Ainsi, l'invention se rapporte à une composition capillaire, caractérisée en ce qu'elle contient, dans un milieu cosmétiquement acceptable, un agent structurant insoluble dans ce milieu et formé de particules solides, présentes en une quantité au moins supérieure à la charge pigmentaire volumique critique conférant à la composition un aspect de solide déformable dans lequel le milieu est emprisonné, cet agent étant apte à s'éliminer du cheveu au moyen d'un diluant.

L'invention a aussi pour objet l'utilisation d'un agent structurant dans une composition capillaire pour lui conférer un aspect de solide déformable, cet agent étant formé de particules solides présentes en une quantité au moins supérieure à la charge pigmentaire volumique critique et étant apte à être éliminé au moyen d'un diluant.

Selon l'invention, l'agent structurant peut être formé d'un ou plusieurs types de particules.

L'un des avantages de cette texture solide est que la composition de l'invention ne risque pas de s'échapper de son conditionnement notamment lors de son transport. Par ailleurs cette composition est très facilement préhensible et ne s'écoule pas entre les doigts. Son dosage est beaucoup plus simple que celui des compositions liquides.

Un autre avantage de cette texture est un excellent confort à l'application. En particulier, on ne constate aucune coulure de la composition, contrairement aux compositions classiques, risquant d'irriter notamment le visage et les yeux. L'absence de coulure est très appréciée dans le cas des permanentes et colorations, ainsi que pour les shampooings destinés aux enfants.

Selon l'invention, la composition se présente sous l'aspect d'un solide déformable, sec, ne tâchant pas et ressemblant à de la guimauve (voir le document US-A-3 682 659 pour la consistance de la guimauve). Ce solide peut être modelé comme de la pâte à modeler pour enfants. Il peut être rompu facilement à la main afin de ne prélever que la quantité nécessaire de produit. En particulier, cette composition peut être conditionnée sous forme de monodose et par exemple sous forme de petits cubes ou de berlingots.

Grâce aux particules de l'invention, il est notamment possible d'obtenir une structure homogène (solide déformable) pour des constituants conduisant normalement à des phases distinctes (constituants non miscibles par exemple huile, eau).

En vue d'obtenir un solide au toucher agréable et doux, il est souhaitable d'utiliser des particules ayant une granulométrie moyenne de 1 µm à 300 µm, par exemple de 5 µm à 200 µm et de préférence de 10µm à 100µm et mieux de 15µm à 40 µm.

Afin de conférer à la composition de l'invention un aspect aéré et léger, on utilise avantageusement des particules ayant une densité inférieure à 0,09 et, mieux, inférieure à 0,06 et, encore mieux, inférieure à 0,04.

En vue d'obtenir cette faible densité, on utilise avantageusement des particules creuses remplies d'un gaz. Ce gaz peut être de l'air, de l'azote, de l'isobutane, de l'isopentane, etc.

Selon une autre caractéristique avantageuse de l'invention, les particules se présentent sous forme de billes. Il est toutefois possible d'utiliser des particules ayant la forme de fibres ou d'aiguilles.

Ces particules peuvent être réalisées en différents matériaux inertes ne réagissant pas chimiquement avec le milieu ou support cosmétiquement acceptable ; en particulier ces particules ne réagissent pas avec les huiles, les tensioactifs, l'eau et les différents autres constituants de la composition, tels que les actifs.

L'agent texturant de l'invention présente la particularité de s'éliminer facilement des cheveux par simple dilution. Il joue en fait le rôle de véhicule ou de réservoir pour le support cosmétique. Il permet, en outre, de récupérer le support et notamment le ou les actifs, emprisonnés dans le solide déformable, lorsque c'est nécessaire par simple dilution à l'eau. Ceci est probablement dû au fait que le support cosmétique est logé dans les espaces interparticulaires du solide et non dans les particules.

Outre l'eau, on peut utiliser comme diluant de l'eau additionnée d'un ou plusieurs solvants polaires cosmétiquement acceptables comme les alcools inférieurs (isopropanol, éthanol), le propylène glycol, d'un ou plusieurs tensioactifs. On peut aussi utiliser une eau chargée en sels.

Comme critère de choix de l'agent texturant, on peut réaliser le test suivant :
- ajout de particules déterminées dans de l'eau contenant un colorant classiquement utilisé dans le domaine capillaire tel que le HC Blue 2, jusqu'à l'obtention d'une pâte colorée,
- versement d'une goutte d'eau sur la pâte.

Lorsque la pâte au point d'impact de la goutte d'eau est beaucoup plus claire que le reste de la pâte, cela signifie que les particules considérées sont candidates comme agent texturant. Inversement, lorsque la pâte au point d'impact ne s'est pas décolorée, les particules considérées ne sont nullement appropriées.

Les particules inertes sont avantageusement réalisées en verre ou en matériaux thermoplastiques comme les polyamides tels que le Nylon, les polymères ou copolymères d'acrylonitrile, de chlorure de vinylidène, de chlorure de vinyle et/ou de monomère acrylique ou styrénique, éventuellement expansés. Le monomère acrylique est par exemple un acrylate ou méthacrylate de méthyle ou d'éthyle. Le monomère styrénique est par exemple l'α-méthyl-styrène ou le styrène.

Comme particules de verre utilisables dans l'invention, on peut citer les billes de verres creuses vendues par la société 3M sous la référence Scotchlite Glass Bubbles S 22. 95 % de ces billes ont un diamètre inférieur à 74 µm.

Comme particules de Nylon, on peut utiliser les particules d"Orgasol" vendues par la société Atochem. Ces particules sont des sphères pleines, poreuses, de diamètre allant de 5 µm à 60 µm.

De préférence, les particules sont des particules creuses déformables d'un copolymère, expansé de chlorure de vinylidène et d'acrylonitrile ou de chlorure de vinylidène, d'acrylonitrile et de méthacrylate. On peut par exemple utiliser un terpolymère contenant : de 0 % à 60% de motifs dérivés du chlorure de vinylidène, de 20 % à 90 % de motifs dérivés d'acrylonitrile et de 0 % à 50 % de motifs dérivés d'un monomère acrylique ou styrénique, la somme des pourcentages (en poids) étant égale à 100. Ces particules peuvent être sèches ou hydratées et sont par exemple celles vendues sous la marque EXPANCEL par la société Nobel Casco et en particulier sous les références 551 DE 12 (granulométrie d'environ 12 µm et masse volumique d'environ 40), 551 DE 20 (granulométrie d'environ 30 µm et masse volumique d'environ 65), 551 DE 50 (granulométrie d'environ 40 µm), 461 DE 50 et 642 WE 50 de 50 µm de granulométrie environ, 551 DE 80 (granulométrie de 80 µm environ).

On peut aussi utiliser des particules de ce même terpolymère ayant une granulométrie d'environ 18 µm et une masse volumique d'environ 60 kg/m³ à 80 kg/m³ ou encore de granulométrie d'environ 34 µm et de masse volumique d'environ 20.

On peut encore utiliser des particules de polymère de chlorure de vinylidène et d'acrylonitrile ou de chlorure de vinylidène, d'acrylonitrile et de méthacrylate non expansé comme celles vendues sous la marque EXPANCEL avec la référence 551 DU 10 (granulométrie d'environ 10 µm) ou 461 DU 15 (granulométrie d'environ 15).

Comme autres particules creuses polymériques utilisables dans l'invention, on peut encore citer les polymères et les copolymères obtenus à partir des esters ou acides, itaconique, citraconique, maléique, fumarique, de l'acétate ou lactate de vinyle. (Voir à cet effet le document JP-A-2-112304).

En revanche, les particules d'amidon de maïs, de silice pyrogénée, de polyéthylène, de polyuréthanne ou de polyester non expansé ne permettent pas d'obtenir une composition solide qui s'élimine bien des cheveux lors du rinçage.

Il est certes connu de modifier la viscosité d'un milieu liquide avec des particules solides (voir à ce sujet l'article Elsevier Sequoia, 1992, Progress in Organic Coatings, 21, p.255-267, de A. Toussaint "Choice of rheological model for steady flow : application to industrial concentrated suspensions") mais personne jusqu'à ce jour n'a ni décrit ni suggéré d'utiliser le produit solide, obtenu à partir d'une certaine concentration des particules, dans le domaine capillaire pour stocker le milieu dans lequel sont dispersées les particules.

Autrement dit, l'obtention ou non du solide déformable est liée à la quantité d'agent structurant utilisée dans la composition ; à partir d'une certaine quantité de particules, appelée charge pigmentaire volumique critique et notée C.P.V.C., on note une augmentation brusque de la viscosité du milieu. La C.P.V.C. est fonction du milieu et de la nature des particules elle doit donc être déterminée à chaque fois. Sa détermination ne pose aucun problème pour l'homme du métier. On peut, par exemple, utiliser la méthode officielle ASTM pour déterminer la C.P.V.C.

La composition de l'invention peut consister en un shampooing, un après shampooing, un produit de mise en forme, un agent pour permanenter ou pour défriser les cheveux, une composition de coloration ou de décoloration par voie directe ou d'oxydation. Selon l'application particulière envisagée, la composition de l'invention peut contenir au moins un actif choisi parmi les détergents, les colorants, les agents de permanente, les polymères, les cations.

Dans le cas d'une coloration par oxydation, la composition de l'invention peut être conditionnée dans deux récipients distincts contenant chacun un solide déformable, l'un emprisonnant la composition tinctoriale contenant les précurseurs de colorants d'oxydation (base + coupleur), l'autre emprisonnant l'agent oxydant. Ces deux solides peuvent être mélangés à la main, juste avant emploi. Le mélange est ensuite appliqué sur les cheveux humides. Puis, on laisse agir comme toute composition de coloration classique et enfin on rince les cheveux.

Par ailleurs, l'introduction de ces particules dans des compositions de permanentes permet de diminuer notablement leur odeur.

L'invention a encore pour objet l'utilisation de la composition définie précédemment pour traiter les fibres kératiniques et notamment les cheveux.

La composition de l'invention est avantageusement appliquée sur des fibres kératiniques mouillées. Elle peut cependant être utilisée sur des cheveux secs après l'avoir préalablement diluée à l'eau.

Ainsi, l'invention a encore pour objet un procédé de traitement des fibres kératiniques notamment mouillées, consistant à appliquer sur les fibres mouillées une composition telle que définie ci-dessus, puis à rincer lesdites fibres. Plus spécialement, l'invention se rapporte encore à un procédé pour colorer, décolorer, permanenter, laver et/ou conditionner les fibres kératiniques, consistant à appliquer sur les fibres, en présence d'eau, une composition telle que définie plus avant, puis à rincer lesdites fibres.

L'invention a encore pour objet un procédé pour délivrer un actif sur des fibres kératiniques, consistant à emprisonner l'actif dans un solide déformable comportant un milieu cosmétiquement acceptable et un agent structurant insoluble dans ce milieu, formé de particules solides ; à appliquer une partie au moins du solide déformable sur les fibres, en présence d'eau, pour y libérer l'actif, puis à éliminer l'agent structurant par rinçage des fibres.

La composition de l'invention contient, outre les particules texturantes et les actifs, tous les constituants classiquement utilisés dans les compositions capillaires. Ces constituants sont notamment des huiles minérales, végétales, synthétiques ou siliconées, de l'eau, des solvants alcooliques, des filtres, des parfums, des tensioactifs, des polymères, des conservateurs, des antioxydants, des agents régulateurs de pH, des séquestrants, des charges, etc.

Les exemples ci-après sont donnés à titre illustratif et non limitatif en vue de mieux faire ressortir les caractéristiques de l'invention. Dans les exemples, MA signifie matière active et OE signifie mole d'oxyde d'éthylène.

### Exemple 1 : Shampooing

- Lauryl éther sulfate de sodium à 2,2 OE vendu sous le nom d'Empicol ESB 3/FG par la Société Albright et Wilson à 28 % MA 15 % MA
- Cocoylbetaïne 3 % MA
- Polydiméthylsiloxane vendu sous le nom de Silbione huile 70 047V500000 par la Société Rhône Poulenc 2,7 %
- Chlorure de sodium 11 %
- Scotchlite Glass Bubbles S22 de la Société 3M (sphères creuses de verre) 25 %
- Conservateur, parfum qs
- Eau qsp 100 %
- pH ajusté par HCI à 8

On obtient une pâte blanche lisse facilement modelable, d'application facile, de rinçage satisfaisant.

### Contre exemple 1 : Shampooing

- Lauryl éther sulfate de sodium et de magnésium 80/20 à 4 moles OE vendu sous le nom d'Empicol BSD par Albright et Wilson 6 % MA
- Alkyl polyglucoside en solution aqueuse à 60 % vendu sous le nom d'Oramix CG110 par la Société Seppic 15 % MA
- Diuréthane d'alcools (C16/C18) oxyéthyléné et oxypropyléné vendu sous le nom de Dapral T212 par la Société Akzo 3 %
- Amidon de maïs vendu sous le nom de C*Plus 05391 par la Société Cevestar 45 %
- Conservateur, parfum qs
- Eau qsp 100 %
- pH ajusté par NaOH à 5

On obtient une pâte blanche modelable, collante, d'application difficile, d'élimination longue et de toucher rêche.

### Exemple 2 : Shampooing

- Lauryl éther sulfate de sodium à 2,2 OE vendu sous le nom d'Empicol ESB 3/FL par la Société Albright et Wilson à 28 % MA 13 % MA
- Cocoylbétaïne 3 % MA
- Polydiméthylsiloxane vendu sous le nom de Silbione huile 70 047V500000 par la Société Rhône Poulenc 3 %
- Monoisopropanolamide d'acide de coprah 4 %
- Microsphères expansées de copolymère chlorure de vinyle/acrylonitrile contenant de l'isobutane vendu sous le nom d'Expancel 551 DE 20 8 %
- Conservateur, parfum qs
- Eau qsp 100 %
- pH ajusté par NaOH à 7,5
On obtient une pâte blanche non collante, lisse, facilement modelable, d'application et d'élimination faciles et douce au toucher. Le pouvoir moussant de ce shampooing est comparable à ceux des shampooings connus.

### Contre exemple 2 : Shampooing

- Mono sulfosuccinate d'alcool laurique oxyéthyléné (30 E) vendu sous le nom de Rewopol SB FA 30 K4 par la Société Rewo 3 % MA
- Lauryl éther sulfate de sodium à 2,2 OE vendue sous le nom d'Empicol ESB 3/FG 8,4 % MA
- Disodium cocoamphodiacétate 2 % MA
- Mélange de glycérides de palme (200 OE) et de coprah (7 OE) 80/20 en suspension aqueuse à 70 % vendu sous le nom de Rewoderm LIS 80 par la Société Rewo 2,1 %
- Silice pyrogénée à caractère hydrophobe vendue sous le nom d'Aérosil R 972 par la Société Degussa Silices 24 %
- Conservateurs, parfum qs
- Eau qsp 100 %
- pH ajusté par HCI à 7,5

On obtient une pâte blanche, légèrement gélifiée, malléable, d'application et d'élimination difficiles. Le toucher est rêche.

### Exemple 3 : Shampooing

- Lauryl éther sulfate de sodium à 2,2 OE vendu sous le nom d'Empicol ESB 3/FG 11 %
- Cocoylbétaïne 1,8 %
- Monoisopropanolamide d'acide de coprah 2,3 %
- Microsphères de nylon 6 sur arvosil, vendues sous le nom d'Orgasol 1002 D Nat. COS. par la Société Atochem 44 %
- Conservateur, parfum qs
- Eau qsp 100 %
- pH ajusté par HCI à 7,5

On obtient une pâte blanche modelable, douce, d'application facile. Le rinçage est facile. Le toucher est doux.

### Exemple 4 : Produit de coloration directe

- Alcool laurique oxyéthyléné 12 OE 10 %
- Dérivé de cellulose 1,5 %
- Expancel 551 DE 20 9 %
- Colorants 2 %
- Eau qsp 100 %
- Acide citrique qsp pH 6

La pâte obtenue est solide, douce à l'application, d'étalement et d'élimination à l'eau aisées. De plus, la pâte est colorée de façon moins intense que les produits de coloration classiques tout en assurant une coloration efficace des cheveux, ce qui permet de moins effrayer les utilisateurs.

### Exemple 5 : Produit de coloration directe

- Alcool laurique oxyéthyléné 12 OE 10 %
- Billes de verre (Scotchlite Glass Bubbles S 22) 35 %
- Dérivé de cellulose 1,5 %
- Colorants 2 %
- Eau qsp 100 %
- Acide citrique qsp pH 6

La pâte obtenue est solide, modelable, et d'élimination facile à l'eau.

### Exemple 6 : Coloration directe

- Alcool laurique oxyéthyléné 12 OE 10 %
- Orgasol 2002 U.D Nat COS (microsphères de Nylon 12) 42 %
- Dérivé de cellulose 1,9 %
- Colorants 2 %
- Eau qsp 100 %
- Acide citrique qsp pH 6

La pâte obtenue est solide, modelable, et d'élimination facile à l'eau.

Dans les exemples 4, 5 et 6, les colorants utilisés sont des colorants classiquement employés en coloration directe, et notamment :
- HC Blue 2
- HC Red 3
- Basic Blue 99.

### Exemple 7 : Coloration d'oxydation

*Phase 1 en pâte contenant les précurseurs de colorants d'oxydation :*
- Lauryl sulfate de sodium 20 %
- Carbopol (polycarboxyvinylique) 2 %
- Expancel 551 DE 20 9 %
- Précurseurs de colorant 2 %
- Ammoniaque en solution aqueuse à 20 % de NH₃ 12 %
- Eau qsp 100 %

La phase 1 est un solide modelable, doux.

*Phase 2 oxydante en pâte :*
- Eau oxygénée à 200 volumes 12 %
- Cires auto-émulsionnantes 2 %
- Stabilisants 0,6 %
- Expancel 551 DE 20 9 %
- Eau qsp 100 %
- pH 2

La phase 2 est aussi un solide modelable, doux.

Au moment de l'emploi, on mélange les deux pâtes à la main puis on applique le mélange sur les cheveux mouillés. On laisse agir 20 min, puis on rince à l'eau. La coloration est comparable à celle obtenue classiquement.

### Exemple 8 : Réducteur pâte

On prépare selon l'invention une pâte réductrice pour la déformation permanente des cheveux en procédant au mélange des ingrédients suivants :
- Acide thioglycolique 10 %
- Séquestrant 0,2 %
- Amidopropyl bétaïne d'acide gras de coprah 1,3 %
- Expancel 551 DE 50 6 %
- Parfum 0,5 %
- Ammoniaque qsp pH=9
- Eau déminéralisée qsp 100 %

On applique cette composition au pinceau sur des cheveux mouillés préalablement enroulés sur des bigoudis. On laisse agir 15 min puis on rince abondamment à l'eau.

On applique alors la composition oxydante suivante :
- Eau oxygénée à 200 volumes qs 4,8 %
- Stabilisants 0,006 %
- Alcool oléique à 20 moles d'oxyde d'éthylène 1,5 % MA
- Acide citrique qsp pH =3
- Eau déminéralisée 100 %

On laisse agir la composition oxydante pendant 10 min. On rince à l'eau puis on enlève les bigoudis. Après séchage, on constate que les cheveux présentent de belles boucles avec un bon degré de frisure. Au cours de l'application du réducteur pâte on n'observe aucune "coulure" du réducteur ainsi qu'une moindre odeur au long de la phase de réduction.

On note, en outre, une diminution importante de l'odeur désagréable par rapport aux compositions classiques de permanente.

### Exemple 9 : Réducteur pâte

Dans cet exemple, on procède de la même manière que dans l'exemple 8, si ce n'est qu'on utilise la pâte réductrice suivante :
- Acide thioglycolique 9 %
- Séquestrant 0,2 %
- Amidopropylbétaïne d'acide gras de coprah 1,3 %
- Scotchlite glass Bubbles S 22 (produit de la Société 3M) 30 %
- Parfum 0,5 %
- Bicarbonate d'ammonium 3 %
- Ammoniaque qsp pH =8,5
- Eau déminéralisée qsp 100 %

### Exemple 10 : Fixateur pâte

Après application du réducteur pâte selon les mêmes compositions que les exemples 8 et 9 et le même mode opératoire que dans ces exemples 8 et 9, on applique au pinceau la composition suivante :
- Eau oxygénée à 200 volumes 4,8 %
- Stabilisants 0,06 %
- Chlorure d'oléocétyldiméthyl-ammonium 1,7 %
- Expancel 551 DE 50 6,0 %
- Acide citrique qsp pH =3
- Eau déminéralisée qsp 100 %

On laisse agir 10 min. On rince à l'eau puis on retire les bigoudis. On obtient le même type de résultat que précédemment.

### Exemple 11 ; Après-shampooing

- Chlorure de Behenyl Triméthyl Ammonium à 80 % dans un mélange eau/isopropanol (15/85) 1 %
- Emulsion cationique DC 929 (fer : Dow Corning) 4 %
- Chlorhydrate de chlorhexidine 0,02 %
- P-hydroxybenzoate de méthyle 0,2 %
- EXPANCEL 551 DE 50 8 %
- Eau déminéralisée stérilisée qsp 100 %

**Mode opératoire ;** On applique sur cheveux humides. On laisse poser 2 minutes. On rince. L'élimination est très facile, le démêlage des cheveux est très bon. Les cheveux une fois séchés sont très doux et brillants.

## Revendications

1. Composition capillaire, caractérisée en ce qu'elle contient, dans un milieu cosmétiquement acceptable, un agent structurant insoluble dans ce milieu et formé de particules solides présentes en une quantité au moins supérieure à la charge pigmentaire volumique critique, conférant à la composition un aspect de solide déformable dans lequel le milieu est emprisonné, cet agent étant apte à s'éliminer du cheveu au moyen d'un diluant.

2. Composition selon la revendication 1, caractérisée en ce que l'agent structurant a une granulométrie moyenne de 1 µm à 300 µm.

3. Composition selon la revendication 1 ou 2, caractérisée en ce que l'agent structurant a une granulométrie moyenne allant de 10 µm à 100 µm.

4. Composition selon l'une des revendications précédentes, caractérisée en ce que l'agent structurant a une densité inférieure à 0,09.

5. Composition selon l'une quelconque des revendications précédentes, caractérisée en ce que l'agent structurant a une densité inférieure à 0,04.

6. Composition selon l'une quelconque des revendications précédentes, caractérisée en ce que les particules sont creuses.

7. Composition selon l'une quelconque des revendications précédentes, caractérisée en ce que les particules sont en matériau thermoplastique.

8. Composition selon l'une quelconque des revendications précédentes, caractérisée en ce que les particules sont réalisées en un matériau choisi parmi le verre, les polymères et copolymères de polychlorure de vinylidène, d'acrylonitrile et/ou d'un monomère acrylique ou styrénique, le Nylon.

9. Composition selon l'une quelconque des revendications précédentes, caractérisée en ce que les particules sont des particules creuses de copolymère de chlorure de vinylidène, d'acrylonitrile et/ou d'un monomère acrylique ou styrénique, expansé.

10. Composition selon l'une des revendications précédentes, caractérisée en ce qu'elle contient, en outre, au moins un actif choisi parmi les détergents, les colorants, les agents de permanente, les polymères, les cations.

11. Utilisation de la composition selon l'une quelconque des revendications précédentes pour traiter les fibres kératiniques.

12. Procédé pour traiter les fibres kératiniques, consistant à appliquer sur les fibres une composition selon l'une quelconque des revendications 1 à 10, puis à rincer lesdites fibres.

13. Procédé pour colorer, décolorer, permanenter, laver et/ou conditionner les fibres kératiniques, consistant à appliquer sur les fibres une composition selon l'une quelconque des revendications 1 à 10, puis à rincer lesdites fibres.

14. Utilisation d'un agent structurant dans une composition capillaire pour lui conférer un aspect de solide déformable, cet agent étant formé de particules solides présentes en une quantité au moins supérieure à la charge pigmentaire volumique critique et étant apte à être éliminé au moyen d'un diluant.

15. Utilisation selon la revendication 14, caractérisée en ce que les particules ont une granulométrie moyenne allant de 1 µm à 300 µm.

16. Utilisation selon la revendication 14 ou 15, caractérisée en ce que l'agent structurant a une granulométrie moyenne allant de 10 µm à 100 µm.

17. Utilisation selon l'une des revendications 14 à 16, caractérisée en ce que l'agent structurant a une densité inférieure à 0,09.

18. Utilisation selon l'une des revendications 14 à 17, caractérisée en ce que l'agent structurant a une densité inférieure à 0,04.

19. Utilisation selon l'une des revendications 14 à 18, caractérisée en ce que les particules sont creuses.

20. Utilisation selon l'une des revendications 14 à 19, caractérisée en ce que les particules sont en matériau thermoplastique.

21. Utilisation selon l'une des revendications 14 à 20, caractérisée en ce que les particules sont réalisées en un matériau choisi parmi le verre, les polymères et copolymères de chlorure de vinylidène, d'acrylonitrile et/ou d'un monomère acrylique ou styrénique, le Nylon.

22. Utilisation selon l'une des revendications 14 à 21, caractérisée en ce que les particules sont des particules creuses de copolymère de chlorure de vinylidène, d'acrylonitrile et/ou d'un monomère acrylique ou styrénique, expansé.

23. Utilisation selon l'une des revendications 14 à 22, caractérisée en ce que la composition contient, en outre, au moins un actif choisi parmi les détergents, les colorants, les agents de permanente, les polymères, les cations.

24. Procédé pour délivrer un actif sur des fibres kératiniques, consistant à emprisonner l'actif dans un solide déformable comportant un milieu cosmétiquement acceptable et un agent structurant insoluble dans ce milieu, formé de particules solides présentes en une quantité au moins supérieure à la charge pigmentaire volumique critique ; à appliquer une partie au moins du solide déformable sur les fibres, en présence d'eau, pour y libérer l'actif puis à éliminer l'agent structurant par rinçage des fibres.

25. Procédé selon la revendication 24, caractérisé en ce que l'agent structurant a une granulométrie moyenne de 1 µm à 300 µm.

26. Procédé selon la revendication 24 ou 25, caractérisé en ce que l'agent structurant a une granulométrie moyenne allant de 10 µm à 100 µm.

27. Procédé selon l'une des revendications 24 à 26, caractérisé en ce que l'agent structurant a une densité inférieure à 0,09.

28. Procédé selon l'une des revendications 24 à 27, caractérisé en ce que les particules sont creuses.

29. Procédé selon l'une des revendications 24 à 28, caractérisé en ce que les particules sont réalisées en un matériau choisi parmi le verre, les polymères et copolymères de polychlorure de vinylidène, le Nylon.

30. Procédé selon l'une des revendications 24 à 29, caractérisé en ce que les particules sont des particules creuses à base de polychlorure de vinylidène expansé.

31. Procédé selon l'une des revendications 24 à 30, caractérisé en ce que l'actif est choisi parmi les détergents, les colorants, les agents de permanente.

## Patentansprüche

1. Zusammensetzung für das Haar, dadurch gekennzeichnet, daß sie in einem kosmetisch akzeptablen Medium ein in diesem Medium unlösliches Strukturierungsmittel enthält, das aus festen Partikeln gebildet wird, die in einer Menge vorliegen, die zumindest über der kritischen, volumenbezogenen Pigmentkonzentration liegt, und die der Zusammensetzung das Aussehen eines verformbaren Feststoffs verleihen, in dem das Medium eingeschlossen ist, wobei das Mittel über ein Verdünnungsmittel aus dem Haar entfernt werden kann.

2. Zusammensetzung nach Anspruch 1, dadurch gekennzeichnet, daß das Strukturierungsmittel eine mittlere Partikelgröße von 1 µm bis 300 µm aufweist.

3. Zusammensetzung nach Anspruch 1 oder 2, dadurch gekennzeichnet, daß das Strukturierungsmittel eine mittlere Partikelgröße von 10 µm bis 100 µm aufweist.

4. Zusammensetzung nach einem der vorhergehenden Ansprüche, dadurch gekennzeichnet, daß das Strukturierungsmittel eine Dichte unter 0,09 aufweist.

5. Zusammensetzung nach einem der vorhergehenden Ansprüche, dadurch gekennzeichnet, daß das Strukturierungsmittel eine Dichte unter 0,04 aufweist.

6. Zusammensetzung nach einem der vorhergehenden Ansprüche, dadurch gekennzeichnet, daß die Partikel hohl sind.

7. Zusammensetzung nach einem der vorhergehenden Ansprüche, dadurch gekennzeichnet, daß die Partikel aus einem thermoplastischen Material bestehen.

8. Zusammensetzung nach einem der vorhergehenden Ansprüche, dadurch gekennzeichnet, daß die Partikel aus einem Material hergestellt sind, das unter Glas, Polymeren und Copolymeren von Vinylidenchlorid, Acrylnitril und/oder einem Acryl- oder Styrolmonomer und Nylon ausgewählt ist.

9. Zusammensetzung nach einem der vorhergehenden Ansprüche, dadurch gekennzeichnet, daß die Partikel expandierte Hohlpartikel eines Copolymers von Vinylidenchlorid, Acrylnitril und/oder einem Acryl- oder Styrolmonomer sind.

10. Zusammensetzung nach einem der vorhergehenden Ansprüche, dadurch gekennzeichnet, daß sie ferner mindestens einen Wirkstoff enthält, der unter den reinigenden Substanzen, Färbemitteln, Mitteln für Dauerwellen, Polymeren und Kationen ausgewählt ist.

11. Verwendung der Zusammensetzung nach einem der vorhergehenden Ansprüche zur Behandlung von Keratinfasern.

12. Verfahren zur Behandlung von Keratinfasern, das darin besteht, auf die Fasern eine Zusammensetzung nach einem der Ansprüche 1 bis 11 aufzutragen und die Fasern anschließend zu spülen.

13. Verfahren zum Färben, Entfärben, dauerhaften Verformen und/oder Konditionieren von Keratinfasern, das darin besteht, auf die Fasern eine Zusammensetzung nach einem der Ansprüche 1 bis 11 aufzutragen und die Fasern anschließend zu spülen.

14. Verwendung eines Strukturierungsmittels in einer Zusammensetzung für das Haar, um ihr das Aussehen eines verformbaren Feststoffs zu verleihen, wobei das Mittel aus festen Partikel gebildet wird, die in einer Menge vorliegen, die zumindest über der kritischen, volumenbezogenen Pigmentkonzentration liegt, und über ein Verdünnungsmittel entfernt werden kann.

15. Verwendung nach Anspruch 15, dadurch gekennzeichnet, daß die Partikel eine mittlere Partikelgröße von 1 µm bis 300 µm aufweisen.

16. Verwendung nach Anspruch 15 oder 16, dadurch gekennzeichnet, daß die Partikel eine mittlere Partikelgröße von 10 µm bis 100 µm aufweisen.

17. Verwendung nach einem der Ansprüche 15 bis 17, dadurch gekennzeichnet, daß das Strukturierungsmittel eine Dichte unter 0,9 aufweist.

18. Verwendung nach einem der Ansprüche 15 bis 18, dadurch gekennzeichnet, daß das Strukturierungsmittel eine Dichte unter 0,4 aufweist.

19. Verwendung nach einem der Ansprüche 15 bis 19, dadurch gekennzeichnet, daß die Partikel hohl sind.

20. Verwendung nach einem der Ansprüche 15 bis 20, dadurch gekennzeichnet, daß die Partikel aus einem thermoplastischen Material bestehen.

21. Verwendung nach einem der Ansprüche 15 bis 21, dadurch gekennzeichnet, daß die Partikel aus einem Material hergestellt sind, das unter Glas, Polymeren und Copolymeren von Vinylidenchlorid, Acrylnitril und/oder einem Acryl- oder Styrolmonomer und Nylon ausgewählt ist.

22. Verwendung nach einem der Ansprüche 15 bis 22, dadurch gekennzeichnet, daß die Partikel expandierte Hohlpartikel eines Copolymers von Vinylidenchlorid, Acrylnitril und/oder einem Acryl- oder Styrolmonomer sind.

23. Verwendung nach einem der Ansprüche 16 bis 23, dadurch gekennzeichnet, daß die Zusammensetzung ferner mindestens einen Wirkstoff enthält, der unter den reinigenden Substanzen, Färbemitteln, Mitteln für Dauerwellen, Polymeren und Kationen ausgewählt ist.

24. Verfahren, um einen Wirkstoff auf die Keratinfasern zu bringen, das darin besteht, einen Wirkstoff in einem deformierbaren Feststoff einzuschleißen, der ein kosmetisch akzeptables Medium und ein in diesem Medium unlösliches Strukturierungsmittel enthält, das aus festen Partikeln gebildet wird, die in einer Menge vorliegen, die zumindest über der kritischen, volumenbezogenen Pigmentkonzentration liegt, in Gegenwart von Wasser mindestens einen Teil des deformierbaren Feststoffs auf die Fasern aufzubringen, um den Wirkstoff freizusetzen, und dann durch Spülen der Fasern das Strukturierungsmittel zu entfernen.

25. Verfahren nach Anspruch 25, dadurch gekennzeichnet, daß das Strukturierungsmittel eine mittlere Partikelgröße von 1 µm bis 300 µm aufweist.

26. Verfahren nach Anspruch 25 oder 26, dadurch gekennzeichnet, daß das Strukturierungsmittel eine mittlere Partikelgröße von 10 µm bis 100 µm aufweist.

27. Verfahren nach einem der Ansprüche 25 bis 27, dadurch gekennzeichnet, daß das Strukturierungsmittel eine Dichte unter 0,09 aufweist.

28. Verfahren nach einem der Ansprüche 25 bis 28, dadurch gekennzeichnet, daß die Partikel hohl sind.

29. Verfahren nach einem der Ansprüche 25 bis 29, dadurch gekennzeichnet, daß die Partikel aus einem Material hergestellt sind, das unter Glas, Polymeren und Copolymeren von Polyvinylidenchlorid und Nylon ausgewählt ist.

30. Verfahren nach einem der Ansprüche 25 bis 30, dadurch gekennzeichnet, daß die Partikel Hohlpartikel auf der Basis von expandiertem Polyvinylidenchlorid sind.

31. Verfahren nach einem der Ansprüche 25 bis 31, dadurch gekennzeichnet, daß der Wirkstoff unter den reinigenden Substanzen, Färbemitteln und Mitteln für Dauerwellen ausgewählt ist.

## Claims

1. Hair composition, characterized in that it contains, in a cosmetically acceptable medium, a structuring agent insoluble in this medium and formed of solid particles present in an amount at least greater than the critical pigment charge volume, which imparts a deformable solid appearance to the composition in which the medium is contained, this agent being capable of being removed from the hair using a diluent.

2. Composition according to Claim 1, characterized in that the structuring agent has a mean particle size of from 1 µm to 300 µm.

3. Composition according to Claim 1 or 2, characterized in that the structuring agent has a mean particle size ranging from 10 µm to 100 µm.

4. Composition according to one of the preceding claims, characterized in that the structuring agent has a density of less than 0.09.

5. Composition according to any one of the preceding claims, characterized in that the structuring agent has a density of less than 0.04.

6. Composition according to any one of the preceding claims, characterized in that the particles are hollow.

7. Composition according to any one of the preceding claims, characterized in that the particles are made of a thermoplastic material.

8. Composition according to any one of the preceding claims, characterized in that the particles are made of a material chosen from glass, polymers and copolymers of poly(vinylidene chloride), of acrylonitrile and/or of an acrylic or styrene monomer, and nylon.

9. Composition according to any one of the preceding claims, characterized in that the particles are hollow particles of a copolymer of vinylidene chloride, of acrylonitrile and/or of an acrylic or styrene monomer, which is expanded.

10. Composition according to one of the preceding claims, characterized in that it additionally contains at least one active agent chosen from detergents, dyes, permanent-waving agents, polymers and cations.

11. Use of the composition according to any one of the preceding claims for treating keratin fibres.

12. Process for treating keratin fibres, consisting in applying a composition according to any one of Claims 1 to 11 to the fibres and then in rinsing the said fibres.

13. Process for dyeing, bleaching, permanent-waving, washing and/or conditioning keratin fibres, consisting in applying a composition according to any one of Claims 1 to 11 to the fibres and then in rinsing the said fibres.

14. Use of a structuring agent in a hair composition in order to impart a deformable solid appearance thereto, this agent being formed of solid particles present in an amount at least greater than the critical pigment charge volume and being capable of being removed using a diluent.

15. Use according to Claim 14, characterized in that the particles have a mean particle size ranging from 1 µm to 300 µm.

16. Use according to Claim 14 or 15, characterized in that the structuring agent has a mean particle size ranging from 10 im to 100 im.

17. Use according to one of Claims 14 to 16, characterized in that the structuring agent has a density of less than 0.09.

18. Use according to one of Claims 14 to 17, characterized in that the structuring agent has a density of less than 0.04.

19. Use according to one of Claims 14 to 18, characterized in that the particles are hollow.

20. Use according to one of Claims 14 to 19, characterized in that the particles are made of a thermoplastic material.

21. Use according to one of Claims 14 to 20, characterized in that the particles are made of a material chosen from glass, polymers and copolymers of vinylidene chloride, of acrylonitrile and/or of an acrylic or styrene monomer, and nylon.

22. Use according to one of Claims 14 to 21, characterized in that the particles are hollow particles of a copolymer of vinylidene chloride, of acrylonitrile and/or of an acrylic or styrene monomer, which is expanded.

23. Use according to one of Claims 14 to 22, characterized in that the composition additionally contains at least one active agent chosen from detergents, dyes, permanent-waving agents, polymers and cations.

24. Process for delivering an active agent onto keratin fibres, consisting in enclosing the active agent in a deformable solid containing a cosmetically acceptable medium and a structuring agent insoluble in this medium, formed of solid particles present in an amount at least greater than the critical pigment charge volume; in applying at least some of the deformable solid to the fibres, in the presence of water, in order to release the active agent in it and then in removing the structuring agent by rinsing the fibres.

25. Process according to Claim 24, characterized in that the structuring agent has a mean particle size of from 1 µm to 300 µm.

26. Process according to Claim 24 or 25, characterized in that the structuring agent has a mean particle size ranging from 10 µm to 100 µm.

27. Process according to one of Claims 24 to 27, characterized in that the structuring agent has a density of less than 0.09.

28. Process according to one of Claims 24 to 27, characterized in that the particles are hollow.

29. Process according to one of Claims 24 to 28, characterized in that the particles are made of a material chosen from glass, polymers and copolymers of poly(vinylidene chloride), and nylon.

30. Process according to one of Claims 24 to 29, characterized in that the particles are hollow particles based on expanded poly(vinylidene chloride).

31. Process according to one of Claims 24 to 30, characterized in that the active agent is chosen from detergents, dyes and permanent-waving agents.
